(19) **Europäisches Patentamt / European Patent Office / Office européen des brevets**

(11) **EP 1 438 024 B1**

(12) **EUROPEAN PATENT SPECIFICATION**

(45) Date of publication and mention
of the grant of the patent:
**13.06.2007 Bulletin 2007/24**

(51) Int Cl.:
**_A61K 9/16_** (2006.01)  **_A61K 9/20_** (2006.01)
**_C08J 3/12_** (2006.01)

(21) Application number: **02773232.0**

(22) Date of filing: **23.08.2002**

(86) International application number:
**PCT/US2002/026768**

(87) International publication number:
**WO 2003/020244 (13.03.2003 Gazette 2003/11)**

(54) **PROCESS FOR DISPERSING A FLUID IN SOLID PARTICLES**

VERFAHREN ZUM DISPERGIEREN EINES FLUIDUMS IN FESTSTOFFPARTIKELN

PROCEDE DE DISPERSION D'UN FLUIDE DANS DES PARTICULES SOLIDES

(84) Designated Contracting States:
**AT BE BG CH CY CZ DE DK EE ES FI FR GB GR
IE IT LI LU MC NL PT SE SK TR**

(30) Priority: **04.09.2001 US 317404 P**
**16.07.2002 US 396619 P**

(43) Date of publication of application:
**21.07.2004 Bulletin 2004/30**

(73) Proprietor: **DOW GLOBAL TECHNOLOGIES INC.
Midland, Michigan 48674 (US)**

(72) Inventors:
• **SHESKEY, Paul, J.
Midland, MI 48640 (US)**

• **KEARY, Colin, M.
Midland, MI 48640 (US)**

(74) Representative: **Raynor, John et al
Beck Greener
Fulwood House,
12 Fulwood Place,
London WC1V 6HR (GB)**

(56) References cited:
**EP-A- 0 169 382** **DE-A- 19 844 523**
**DE-A- 19 910 789** **GB-A- 2 355 008**
**US-A- 4 476 145**

EP 1 438 024 B1

**Description**

Background of the Invention

The present invention relates to a process for dispersing a fluid in solid particles, such as a powder, and to a process for preparing tablets.

Processes for dispersing a fluid in solid particles have been known for a long time. Such processes are typically used for coating the solid particles with components comprised in the fluid or for granulating powders. Granulation is widely used in the pharmaceutical industry as a particle size enlargement process. Benefits of granulation include improved flowability of the dried granulated particles, the elimination of hazardous dusts and significant reductions in the volume of material that must be subsequently handled. According to one well-known method a binder is dissolved in a liquid and applied to a powder mass. According to another method a dry binder is mixed with a powder to be granulated and a liquid is added, such as water activates the binder. Unfortunately, in these processes there is the danger of inhomogeneous distribution of the liquid in the powder. Spots of powders with too much liquid result in the formation of large lumps whereas spots of powders with an insufficient amount of liquid do not properly form agglomerates. Accordingly, sophisticated devices are necessary for granulation. Generally the powder to be granulated is vigorously agitated, for example in a high shear granulator, and the liquid is continuously sprayed through spray nozzles or atomizing devices on the agitated powder. However, this process is difficult to define, difficult to control and prone to occasional batch failures. Addition of too much liquid results in a powder which is too wet; addition of an insufficient amount of liquid results in poor granulate quality. In American Pharmaceutical Review, Volume 3, Issue 4, Winter 2000, pages 33-36, "Granulation and Scale-Up Issues in Solid Dosage Form Development", Tony Hlinak explains that fundamental models allowing the prediction of granulation times from measurements of ingredient and binding solutions properties do not exist, forcing the industry toward trial and error approaches to developing the granulation process.

U.S. patent No. 3,725,556 discloses a process for making a tablet which circumvents the conventional granulation prior to compression. The first step of the process consists of spray-drying, after inert gas foaming, a suspension of A) 1- 20 weight percent of a very finely divided silicon oxide or aluminum oxide and B) 60 -98 weight percent of a fine-grained, substantially water-insoluble rice starch or corn starch or an alkaline earth metal phosphate in an aqueous solution of C) 1-20 weight percent of a water-soluble binder. In a second and third step the active ingredient is added to the resulting spray-dried, ungranulated tablet pre-mix and the resulting composition is compressed together with the active agent and a tablet lubricant into pharmaceutical tablets. The resulting tablets readily disintegrate.

Unfortunately, the teaching of U.S. patent No. 3,725,556 does not solve the above-discussed problem that the widely used granulation processes are difficult to control.

U.S. Patent No. 4,476,145 discloses a process wherein dehydrated ingredients, such as a dehydrated vegetable mixture, are coated with foam that has been produced by whipping a hydrophilic binding agent in water. Then uncoated ingredients having a size and density profile different from the foam-coated ingredients, such as breadcrumbs, are mixed with the foam-coated ingredients to produce an agglomerate. Unfortunately, the teaching of U.S. patent No. 4,476,145 does not solve the above-discussed problem that the granulation processes that are widely used in the pharmaceutical industry are difficult to control.

GB-A- 2 355 008 discloses a foamed coating agent for solid or non-aqueous products which protects the product against braking-up and abrasion. The foam is produced by extrusion. Unfortunately, the teaching of GB-A- 2 355 008 does not solve the above-discussed problem that the widely used granulation processes are difficult to control.

It is still highly desirable to provide a granulation process which allows an improved process control.

One object of the present invention is a new process for dispersing a fluid in solid particles. A preferred object of the present invention is to provide a new process for dispersing a fluid in solid particles which does not require the use of an atomizing device. Another preferred object of the invention is a new granulation process. Yet another preferred object of the present invention is to provide a new granulation process wherein the addition of a binder is easy to control. Yet another preferred object of the present invention is to provide a new granulation process wherein dust hazards can be minimized. Yet another preferred object of the present invention is to provide a new granulation process wherein binder efficiency is increased in comparison to conventional processes.

Summary of the Invention

One aspect of the present invention is a process for dispersing a fluid in solid particles which comprises the steps of

    a) contacting a gas with a fluid composition comprising i) from 0.01 to 30 weight percent of a polymer and ii) from 99.99 to 70 weight percent of a liquid diluent, based on the total weight of the polymer i) and the liquid diluent ii), to produce a foam, and

b) contacting the produced foam with solid particles of an average size of less than 2500 micrometers, the weight ratio between the foam and the solid particles being from 1:20 to 1:0.2.

Another aspect of the present invention is a process for preparing a tablet wherein a granular material is produced according to the above-mentioned process and pressed to a tablet.

Short description of the Drawings

Fig. 1 illustrates a foam generating device for producing foam in step a) of the process of the present invention.
Fig. 2 illustrates the release of a drug from tablets prepared according to the invention and according to conventional technology.
Fig. 3 illustrates the release of a drug from another type of tablets prepared according to the invention and according to conventional technology.
Fig. 4 illustrates the release of a drug from yet another type of tablets prepared according to the invention.

Detailed Description of the Invention

The present invention relates to a process for dispersing a fluid in solid particles which comprises the steps a) and b) below. The term "dispersing a fluid in solid particles" as used herein means that the fluid is dispersed in the mass of solid particles. The term as used herein includes various ways of dispersion. For example, the fluid can stay at the surface of the individual solid particles without penetration into the individual solid particles; or the fluid can partially penetrate into the solid particles or can penetrate into some of the particles and remain at the surface of other particles; or the solid particles can absorb the fluid such that the foam penetrates into the solid particles.

As a starting material for step a) of the process of the present invention a fluid composition is prepared which comprises i) a polymer and ii) a liquid diluent. The fluid composition may comprise one or more different polymers. A wide range of polymers is useful. Hydrophilic polymers are preferred. Examples of naturally occurring polymers include gum arabic, xanthan gum, gum karaya, gum tragacanth, gum ghatti, guar gum, exudate gums, seaweed gums, seed gums, microbial gums, carrageenan, dextran, gelatin, alginates, pectins, starches, polysaccharides, such as cellulose ethers or cellulose esters, starch derivatives, guar derivatives or xanthan derivatives. Starch derivatives, guar derivatives or xanthan derivatives are described in more detail in European patent EP 0 504 870 B, page 3, lines 25-56 and page 4, lines 1-30. Useful starch derivatives are for example starch ethers, such as hydroxypropyl starch or carboxymethyl starch. Useful guar derivatives are for example carboxymethyl guar, hydroxypropyl guar, carboxymethyl hydroxypropyl guar or cationized guar. Preferred hydroxypropyl guars and the production thereof is described in U.S patent No. 4,645,812, columns 4-6.

Other examples of useful polymers are homo- or copolymers of ethylene imine, an unsaturated acid, such as acrylic acid or a salt thereof, an unsaturated amide, such as acrylamide, a vinyl polymer, such as vinylalcohol, a vinyl ester, such as vinylacetate, vinylpyrrolidone, vinyloxazolidone, vinylmethyloxazolidone, ethylene sulfonic acid, vinylamine, vinylpyrridine, an alkylglycol, a polyalkylene oxide, such as polyethylene oxide, or an oxyethylene alkylether.

Preferred polymers are cellulose esters or cellulose ethers. Preferred cellulose esters are carboxy-$C_1$-$C_3$-alkyl celluloses, such as carboxymethyl celluloses; or carboxy-$C_1$-$C_3$-alkyl hydroxy-$C_1$-$C_3$-alkyl celluloses, such as carboxymethyl hydroxyethyl celluloses. Preferred cellulose ethers are $C_1$-$C_3$-alkyl celluloses, such as methylcelluloses; $C_1$-$C_3$-alkyl hydroxy-$C_{1-3}$-alkyl celluloses, such as hydroxyethyl methylcelluloses, hydroxypropyl methylcelluloses or ethyl hydroxyethyl celluloses; hydroxy-$C_{1-3}$-alkyl celluloses, such as hydroxyethyl celluloses or hydroxypropyl celluloses; mixed hydroxy-$C_1$-$C_3$-alkyl celluloses, such as hydroxyethyl hydroxypropyl celluloses, or alkoxy hydroxyethyl hydroxypropyl celluloses, the alkoxy group being straight-chain or branched and containing 2 to 8 carbon atoms. Most preferably, the fluid composition comprises a water-soluble cellulose ether, such as a methylcellulose with a methyl molar substitution $DS_{methoxyl}$ of from 0.5 to 3.0, preferably from 1 to 2.5, or a hydroxypropyl methylcellulose with a $DS_{methoxyl}$ of from 0.5 to 3.0, preferably from 1 to 2.5 and a $MS_{hydroxypropoxyl}$ of from 0.05 to 2.0, preferably from 0.1 to 1.5. The viscosity of the cellulose ether generally is from 1 to 100,000 mPa·s, preferably from 3 to 10,000 mPa·s, more preferably from 3 to 5,000 mPa·s, and most preferably from 5 to 200 mPa·s, measured as a 2-wt.% aqueous solution at 20°C using an Ubbelohde viscometer.

Preferably, the polymers are non-crosslinked. Preferably polymer i) forms a self-supporting, coherent film if the polymer i) is in its undiluted stage.

The polymer i) generally has a weight average molecular weight of at least 10,000, preferably at least 12,000, more preferably at least 15,000, most preferably at least 18,000. The preferred upper limit for the weight average molecular weight largely depends on the type of polymer. Generally the weight average molecular weight of the polymer i) is up to 5,000,000, preferably up to 500,000, more preferably up to 100,000. Most preferably, cellulose ethers have a weight average molecular weight of up to 25,000.

The term "liquid diluent" means a diluent that is liquid at normal pressure and 25ºC. The liquid diluent preferably is a monomeric compound or an oligomeric compound with a molecular weight of up to 500, preferably up to 300. Useful organic liquids are alcohols, preferably monofunctional alcohols, such as ethanol; alkenes, alkanes, halogenated alkenes, halogenated alkanes, ethers, esters or oils, such as paraffin oils, animal oils or vegetable oils. Most preferably, the liquid diluent is water. The fluid composition used in step a) of the present invention comprises from 0.01 to 30 percent, preferably from 0.1 to 20 percent, more preferably from 0.5 to 15 percent, and most preferably from 1 to 5 percent of the polymer i) and from 99.99 to 70 percent, preferably from 99.9 to 80 percent, more preferably from 99.5 to 85 percent, and most preferably from 99 to 95 percent of the liquid diluent ii), based on the total weight of the polymer i) and the liquid diluent ii). Generally polymers i) are chosen which have surface-active properties. The above-mentioned polymers, particularly water-soluble cellulose ethers, are useful as a surfactant in a water-based fluid composition used in step a) of the process of the present invention. Preferably, the fluid composition does not comprise a substantial amount of a surfactant other than the polymer i). This means that the fluid composition preferably does not contain a surfactant other than the polymer i) in a sufficient amount to cause foaming of the fluid composition upon contact with a gas. More preferably, the fluid composition does not comprise any amount of a surfactant other than the polymer i). Most preferably, the fluid composition does not comprise a known nonionic, cationic, anionic or amphoteric surfactant, as for example listed in U.S. Patent No. 5,026,735, column 6, lines 47-68 and column 7, lines 1-22.

The fluid composition may contain one or more additional solid or liquid components such as drugs, fillers, pigments flavors or plasticizers. If present, their total amount is generally up to 75 percent, preferably up to 50 percent, more preferably up to 25 percent, based on the total weight of the fluid composition. If the fluid composition comprises a cross-linking agent, its amount preferably is not more than 3 percent, more preferably not more than 1.5 percent, most preferably not more than 0.5 weight percent, based on the weight of the polymer i). Most preferably, the fluid composition is free from cross-linking agents.

A two-phase foam may be composed of an aqueous phase and a gaseous phase or a non-aqueous liquid phase and a gaseous phase. A three-phase foam may comprise, in addition to aqueous and gaseous phases, insoluble solids or immiscible liquids. Such three-phase foams can also contain dissolved solids in the aqueous or immiscible liquid phase or in both liquid phases. Four-phase foams may comprise, in addition to aqueous and gaseous phases, immiscible liquids and insoluble solids. In all foams, any immiscible liquid phase may be present as an oil-in-water or water-in-oil emulsion or as a simple dispersion.

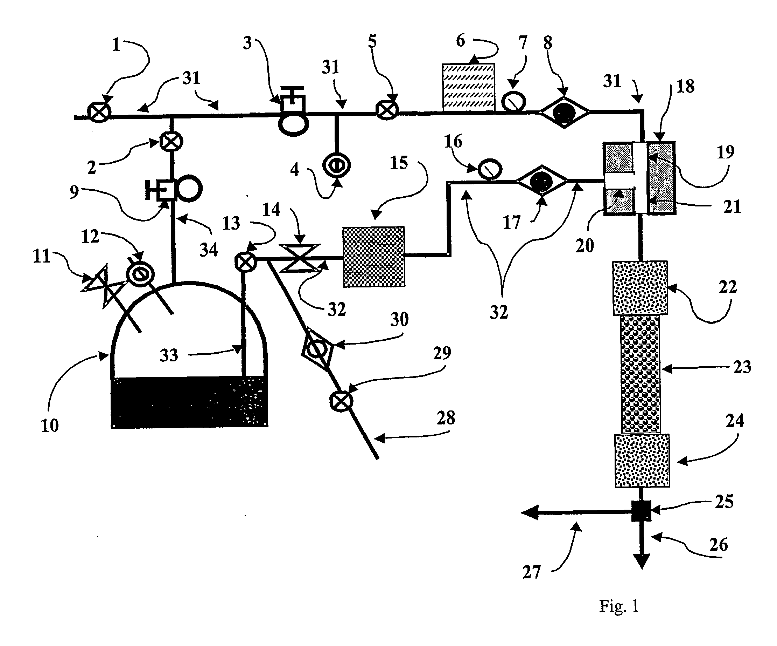

The fluid composition is contacted with a gas, such as oxygen, nitrogen, carbon dioxide or, preferably, air to produce a foam. Preferably a water-based air foam is produced. The term "air foam" is used in its industry-accepted sense to mean a foam made by physically mixing air into a fluid, and thus the term is distinct from chemical or carbon dioxide foam or halocarbon blown foam. The foam can be produced in a known manner by mechanically or physically entraining or dispersing the gas in the fluid composition, for example by pumping the fluid composition to air-aspirating, foam producing equipment. One useful and simple foam generating device is shown in Fig. 1. The gas and the fluid composition are generally contacted at such amounts to produce a foam with an overrun of 50 to 10,000 percent, preferably from 80 to 2,000 percent, more preferably from 100 to 1,500 percent. The overrun is defined below.

$$\text{Overrun (\%)} = [(\text{volume foam} - \text{volume fluid})/\text{volume fluid}] \times 100.$$

The overrun is measured at 25°C and atmospheric pressure.

The produced foam comprises a discontinuous gas phase, preferably an air phase, and a continuous fluid phase, preferably an aqueous phase, comprising the polymer and bound liquid. Generally the lamella or fluid film of the gas bubbles is viscous due to the presence of the polymer. In case the fluid film comprises a hydrophilic polymer such as a cellulose ether, water is retained in the lamella of the gas bubbles. The drainage of the liquid from the lamellae is minimized, reduced or prevented; such a foam is designated as "non-draining foam" in the art. The foam produced in step a) generally has an average bubble diameter in the range of from about 1 micrometer to about 2,000 micrometers, preferably from about 5 micrometers to about 1,000 micrometers, more preferably from about 10 micrometers to about 300 micrometers. It is to be understood that the measurements of the foam diameter generally are not very accurate in view of the dynamic properties of the foam. It has been found that surprisingly high foam qualities can be achieved, particularly if the polymer i) in fluid compositions used for producing the foam is a cellulose ether. The foam quality FQ is given in percent at atmospheric pressure and 25°C and is defined as follows:

$$FQ\ (\%)\ = [\text{gas volume} / (\text{gas volume} + \text{fluid volume}) \times 100].$$

The foam quality can be measured by measuring the foam volume that is produced from a given volume of fluid at atmospheric pressure and 25°C. In step a) of the process of the present invention generally foams with a foam quality of from 52 to 99.9 percent, preferably from 65 to 99.9 percent, more preferably from 85 to 99 percent are produced. Such high foam quality is surprising for "non-draining foams". The foam preferably has a density of up to 0.1 g/cm$^3$.

In step b) of the process of the present invention the foam produced in step a) is contacted with solid particles. The weight ratio between the foam and the solid particles is from 1 : 20 to 1 : 0.2, preferably from 1 : 10 to 1 : 0.5, more preferably from 1 : 5 to 1 : 1. Preferably, the foam and the solid particles are contacted in such ratios that the weight of the above-mentioned polymer i) is from 0.02 to 15, more preferably from 0.1 to 10, most preferably from 0.15 to 8 percent, based on the weight of the solid particles.

The foam can be contacted with a wide variety of solid particles. The particles can be of any shape, such as spherical, elliptic, or fibrous.

According to a preferred embodiment of the invention the solid particles are in the shape of a powder of an average particle size of less than 1000 micrometers, preferably less than 750 micrometers, most preferably less than 500 micrometers. Any powder is useful which traditionally has been coated or agglomerated with a fluid. Preferred classes of useful powders are ingredients of pharmaceutical granules or tablets, ingredients for granules or tablets used in the food or agricultural industry, powders used in ceramic processes, or powdered detergents. Exemplary thereof are pharmaceutical excipients, such as lactose, dicalcium phosphate, microcrystalline cellulose, sugars, minerals, cellulose powder, cellulose fibers, disintegrants, binders, lubricants, colorants, flavorants or combinations thereof or drugs. The foam can be contacted with one or more compounds in powder form. It has been found that the foam lamellae generally break and reform as they pass through the powder during granulation.

According to another preferred embodiment of the present invention the solid particles are in the shape of fibers. It has been found that the process of the present invention is very effective in the production of agglomerates of particles which are typically produced in fibrous shape, such as the above-mentioned cellulose ethers or cellulose esters. By providing an effective process for agglomerating particles in fibrous shape, significant reductions of the dust level of the fibrous material can be achieved.

Advantageously, step b) is conducted in a mixing device, such as a high shear mixing device, a low shear mixing device, a fluidized bed granulator, a roller compactor or a spray dryer. Usually the solid particles are added to the mixing device before it set in operation, but it can also be added later. The contact of the foam with solid particles can be carried out in various ways.

According to one embodiment of the process step b) the mixing device is set into operation after the solid particles and the foam have been fed to the mixing device. This embodiment of step b) is designated as "batch process". Preferably, the solid particles are fed to the mixing device, foam is placed on top of the solid particles and the mixing device is subsequently set into operation. Advantageously, 50 percent or more, more preferably 80 percent or more, most preferably 90 percent or more of the total amount of foam used in step b) is placed on top of the solid particles before the mixing device is set into operation. This embodiment of the process of the present invention is advantageous because it prevents dust emission during the dispersion step b). Surprisingly, is has been found that a uniform dispersion of the foam in the solid particles can be achieved within a very short period after the mixing device has been set in operation, usually within less than 30 seconds, in most cases even within less than 10 seconds, even when the entire amount of foam is placed on top of the solid particles and the mixing device is set into operation only afterwards. This finding is in contrast to a corresponding process wherein a corresponding liquid composition is directly dispersed in the solid particles without formation of foam from the fluid composition. If the liquid is added on top of the solid particles, large lumps are formed and a uniform dispersion of the liquid in the solid particles is impossible.

According to another embodiment of the invention, the process step b) is carried out in the sub-steps of bi) feeding the solid particles and a foam portion to a mixing device, bii) setting the mixing device into operation to disperse the foam portion in the solid particles, biii) stopping the operation of the mixing device, biv) feeding an additional foam portion to the mixing device, bv) setting the mixing device into operation to disperse the foam portion in the solid particles, bvi) stopping the operation of the mixing device, and repeating steps biv) to bvi) several times. This embodiment of step b) is designated as "stepped-batch process".

According to yet another embodiment of the process step b) the solid particles are fed into a mixing device and foam is added continuously or in portions to the mixing device while the mixing device is in operation. This embodiment of step b) is designated as "continuous process".

According to yet another embodiment of the process step b), a part of the foam is added to the solid particles before the mixing device is set in operation and a part of the foam is added continuously or in portions to the solid particles while the mixing device is in operation.

According to the process of the present invention a surprisingly homogeneous dispersion of the polymer and optional other foam components in the solid particles is achieved. Moreover, a simple device can be used for applying the foam to the solid particles, such as a simple tube. Expensive and complex atomizing devices that are commonly used for spraying fine droplets of liquids on solid particles are not necessary. The dispersion of the foam in the solid particles is achieved at a rate which is comparable to or even faster than the dispersion of a corresponding liquid spray in the solid particles. The process of the present invention is also useful for dispersing poorly water-soluble compounds, such as poorly water-soluble drugs, in the solid particles.

Depending on the type of polymer and optional other components in the foam, the components of the foam coat solid particles with or without agglomeration of the solid particles. If the fluid composition does not comprise a binder for the solid particles, the particles are at least partially coated with the foam but usually no agglomeration takes place. This method is particularly useful for coating powders, such as pharmaceutical excipients, with drugs, colorants or other materials in case no agglomeration of the solid particles is desired.

The process of the present invention is particularly useful for agglomerating solid particles and for producing a granular material. If the fluid composition comprises a binder for the solid particles, the particles agglomerate upon contacting the foam with the solid particles. The above-listed polymers can usually act as a polymeric binder. Preferred polymeric binders are cellulose ethers, particularly water-soluble cellulose ethers, such as methylcellulose, hydroxypropyl methylcellulose or hydroxypropyl cellulose. The produced granular material can be subjected to one or more known compounding steps, such as drying, grinding, for example wet-milling or dry-milling, sieving, mixing with optional additional ingredients, for example lubricants, pressing into tablets or combinations thereof. The drying step can be carried out prior to or after the grinding step. Preferably, the produced granular material is dried and processed to a material of an average particle size of from 10 to 10,000 micrometers, preferably from 10 to 5,000 micrometers.

Depending on the composition of the coated particles or the granular material produced according to the process of the present invention, the coated particles or the granular material can be used as-produced, for example as fillings for pharmaceutical capsules, or can be further processed to the desired product, for example it may be pressed to tablets.

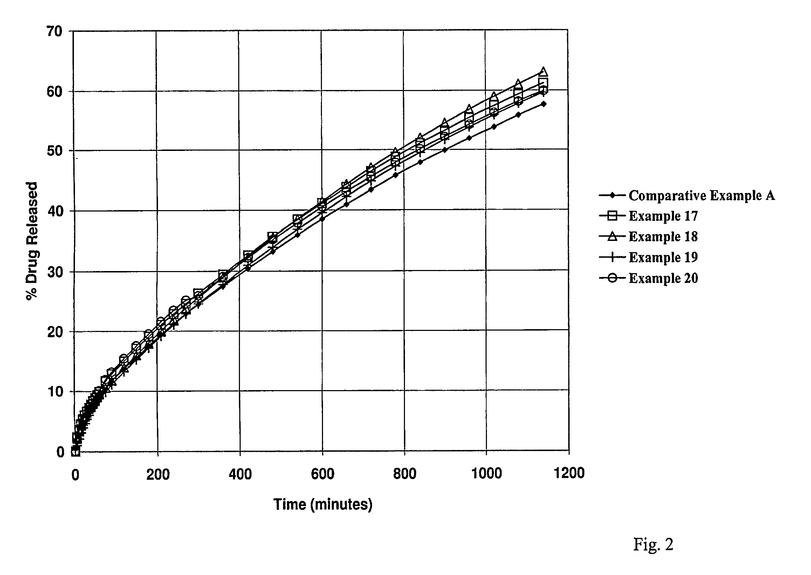

It has been found that the process of the present invention is also useful for preparing pharmaceutical compositions. A fluid composition comprising a drug and/or solid particles comprising a drug is used in the process of the present invention for preparing pharmaceutical compositions. The process of the present invention is particularly useful for preparing a pharmaceutical composition with a controlled drug release. It has been surprisingly found that the agglomeration process of the present invention leads to pharmaceutical compositions with essentially the same drug release profile as a conventional agglomeration process wherein a corresponding liquid is sprayed on corresponding solid particles. As illustrated in Fig. 2, controlled drug release compositions can be prepared according to the process of the present invention wherein the pharmaceutical powder is agglomerated by means of foam in a continuous, stepped-batch or batch process. Surprisingly, it has been found that different embodiments of carrying out the agglomeration process of the present invention, such as the addition of foam continuously, in one batch or in several batches or addition of the foam at different speeds, lead to pharmaceutical compositions with essentially the same drug release profile as a conventional agglomeration process wherein a corresponding liquid is sprayed on a corresponding powder. Due to the present invention, the well-known problems of dispersing a fluid in solid particles can be solved without sacrificing the predictability of the drug release of pharmaceutical compositions prepared from the powder. It has even more surprisingly been found that essentially the same drug release profile is even achieved when a substantially decreased binder level is used in the granulation process of the present invention, as compared to a conventional agglomeration process wherein a corresponding liquid is sprayed on a corresponding powder. Generally at least 5 times less, typically even at least 10 times less binder is necessary in the granulation process of the present invention than in a conventional granulation process using sprayed liquid for obtaining essentially the same drug release in both processes. This means that the process of the present invention makes a much more efficient use of the granulation binder than a conventional granulation process using sprayed liquid.

The present invention is further illustrated by the following examples which should not be construed to limit the scope of the present invention. All parts and percentages are by weight unless otherwise indicated. The alkyl and hydroxyalkyl substitutions of the cellulose ethers indicated in the examples below are measured and calculated according to ASTM D3876. The apparent viscosities indicated in the examples below are measured and normalized to a 2 weight percent aqueous solution using an Ubbelohde viscometer at 20°C.

The compounds listed in Table 1 below are used in the examples.

Table 1

| Designation | Description |
| --- | --- |
| FFL-316 | Fast flow lactose, commercially available from DMV International Pharma and Foremost Farms USA, is a powder component |

(continued)

| Designation | Description |
|---|---|
| Avicel PH102 | Microcrystalline cellulose, commercially available from FMC Corporation, is a powder component |
| K4MP | Hydroxypropyl methylcellulose with a methoxyl substitution of 19-24 percent, a hydroxypropoxyl substitution of 7-12 percent and a viscosity of about 4,000 mPa·s. It is commercially available from The Dow Chemical Company under the Trademark METHOCEL K4MP |
| K3PLV | Hydroxypropyl methylcellulose with a methoxyl substitution of 19-24 percent, a hydroxypropoxyl substitution of 7-12 percent and a viscosity of 3 mPa·s. It is commercially available from The Dow Chemical Company under the Trademark METHOCEL K3PLV. |
| E3P | Hydroxypropyl methylcellulose with a methoxyl substitution of aboout 29 percent, a hydroxypropoxyl substitution of about 9 percent and a viscosity of 3 mPa·s. It is commercially available from The Dow Chemical Company under the Trademark METHOCEL E3PLV. |
| E6PLV | Hydroxypropyl methylcellulose with a methoxyl substitution of 28-30 percent, a hydroxypropoxyl substitution of 7-12 percent and a viscosity of about 6 mPa·s. It is commercially available from The Dow Chemical Company under the Trademark METHOCEL E6PLV |
| Foamed E6PLV solution | Foam prepared from 1% and 5% aqueous solutions of METHOCEL E6PLV cellulose ether, as described in step a) below |
| CELLOSIZE™ QP3L | Hydroxyethyl cellulose with an MS value of about 2, and a viscosity of 215-282 mPa.sec at 25C. |
| SLS | Sodium lauryl sulfate surfactant |
| PVP-K30 | Polyvinylpyrrolidone K30, USP-NF, molecular weight about 40,000 |

Examples 1 - 16

a) Production of the Foam

An aqueous solution containing 1% (weight percent) or 5% (weight percent) of a binder is prepared from a METHOCEL E6PLV cellulose ether. From the aqueous solution a foam is prepared as illustrated in Fig. 1.

Air flows through a tube 31 equipped with ball valves 1, 2 and 5, with pressure regulators and gauge 3 and 9, with a pressure relief valve 4, a mass flow controller 6, a pressure gauge 7 and a check valve 8. The aqueous solution is passed from a pressure vessel 10, which is equipped with a pressure relief valve 12, needle valve 11, air inlet tube 34 and a dip-pipe 33, through a tube 32. Tube 32 is equipped with a ball valve 13, a needle valve 14, an oval gear flow meter 15, a pressure gauge 16 and a check valve 17 and with a water supply line 28, a ball-valve 29 and a check valve 30. The air stream and fluid stream meet in T-piece 18 comprising an air-inlet port 19, a fluid inlet port 20 and a foam outlet port 21. The air stream is dispersed in the water stream by in-line filters 22 and 24 and additionally in packed tube 23 whereby the foam is produced and exits the foam production device via tube 26 or 27 according to the position of 3-way valve 25. The in-line filters used for preparing the foams in the examples have a pore size of 90 micrometers, but generally in-line filters with pore sizes of from 0.5 to 90 micrometers, more preferably from 15 to 90 micrometers are useful for simple foams. For foams containing solids or emulsions, the in-line filters are preferably replaced with strainer elements whose only function is to keep the glass beads in tube 23. Such strainer elements preferably have a nominal pore size of about 440 micrometers. The in-line filters 22 and 24 are connected via a tube 23. The stainless steel tube 23 in the foam production device used in the examples is approximately 25 cm. long by 12.8 cm. external diameter, and is packed with glass beads of 3 mm diameter. Other packed-tube foam generators are described in detail in "A mechanical foam-generator for use in laboratories", by J. F. Fry and R. J. French, J. Appl. Chem., 1, 425-429 (1951). The operation of the foam generating device is known to the skilled artisan.

In Example 1 the incoming air is adjusted to about 60 psig, either by regulating the supply or by air pressure regulators 3 and 9. In Example 1 the flow rate of the fluid is set to 0.23 l/min. as indicated by the oval gear flow meter 15. Any change in foam quality may be made by adjusting air and/or fluid flow rates while ensuring that pressures remain below 60 psig, the set-point for pressure relief valves 4 and 12. The produced foam is designated as foamed E6PLV solution.

b) Powder granulation

1200 g of a powder blend are loaded into a high shear granulator. The powder has an average particle size of less than about 150 micrometers. The composition of the powder blend is listed in Table 2 below. The high shear granulator is commercially available from Glatt Air Techniques Inc. under the designation POWREX. The foamed E6PLV solution which is produced as described in step a) above is fed via a tube of 12.8 mm outside diameter into the granulator.

Three distinct modes of operation of the foam granulation technique are illustrated in the Examples given in Table 2. These modes are: batch, stepped-batch and continuous.

In the batch granulation process, illustrated by Examples 3, 4, 10, and 12 in Table 2 the entire amount of the foam is placed on top of the powder at any convenient foam flow rate before the high shear granulator is set into operation. Then the foam is dispersed in the powder by running the granulator, for example, under the following conditions: room temperature, side chopper blades at 1,800 rpm and the main blade at 300 rpm. After as little as 5 to 60 seconds the foam is homogeneously dispersed in the powder.

In the stepped-batch granulation process, illustrated by Example 11 in Table 2, an aliquot of foam is added to the surface of the powder for 60 seconds at 100 gram/minute foam flow rate while the high shear granulator is stationary. After the addition of each aliquot, the foam is homogeneously dispersed in the powder by running the granulator, for example, at room temperature, side chopper blades at 1,800 rpm and main blade at 300 rpm for as little as 5 to 20 seconds. The granulate is examined visually and manually to determine a qualitative degree of wetness and granularity. The procedure is repeated eight times, that means eight aliquots of foam are added for sixty seconds each at 100 gram/minute flow rate, until an acceptable wetness and granularity are achieved. In Example 11 the qualitative end-point that is familiar to those skilled in the art of high-shear granulation is achieved upon addition of a total of 800 grams of foam. In Example 12 in Table 2, 800 grams of the same foam are added as a single batch to a fresh load of powder and the batch granulation process is followed, as described above. The granulated product produced according to the batch process is qualitatively indistinguishable from the granulated product of Example 11 of Table 2. From a comparison of Examples 11 and 12 the stepped-batch process may be considered as a "learning mode" to be used when formulators first encounter an unfamiliar powder system that has to be granulated. This means that the stepped-batch mode may be used to determine the relative proportions of foam and powder that should be employed in subsequent batch or continuous granulation processes involving the same formulation.

In the continuous granulation process, illustrated by Examples 1, 2, 5-9, and 13-16 of Table 2, the foam is added to the powder while running the granulator, for example, at room temperature, side chopper blades at 1,800 rpm and main blade at 300 rpm. The foam is homogeneously dispersed in the powder as it is being added.

Table 2

| Example | Process type | Powder composition | | | E6PLV solution | Foam | | Granulate |
|---|---|---|---|---|---|---|---|---|
| | | K4MP (%)[1] | FLL-316 (%)[1] | Avicel PH102 (%)[1] | Binder concentration, based on total solution (%) | Addition rate (g/minute) | Addition time (seconds) | Binder level (%)[1] |
| 1 | Continuous | - | 80 | 20 | 1 | 230 | 60 | 0.19 |
| 2 | Continuous | - | 80 | 20 | 5 | 50 | 540 | 1.88 |
| 3 | Batch | - | 80 | 20 | 1 | 300 | 46 | 0.19 |
| 4 | Batch | - | 80 | 20 | 5 | 100 | 240 | 1.67 |
| 5 | Continuous | - | 80 | 20 | 5 | 50 | 540 | 1.88 |
| 6 | Continuous | - | 80 | 20 | 5 | 100 | 260 | 1.81 |
| 7 | Continuous | - | 80 | 20 | 5 | 150 | 180 | 1.88 |
| 8 | Continuous | 30 | 50 | 20 | 1 | 300 | 153 | 0.64 |
| 9 | Continuous | 30 | 50 | 20 | 5 | 100 | 750 | 5.21 |
| 10 | Batch | 30 | 50 | 20 | 1 | 300 | 150 | 0.63 |

(continued)

| Example | Process type | Powder composition | | | E6PLV solution | Foam | | Granulate |
|---|---|---|---|---|---|---|---|---|
| | | K4MP (%)[1] | FLL-316 (%)[1] | Avicel PH102 (%)[1] | Binder concentration, based on total solution (%) | Addition rate (g/minute) | Addition time (seconds) | Binder level (%)[1] |
| 11 | Stepped-Batch | 30 | 50 | 20 | 5 | 100 | 480 | 3.33 |
| 12 | Batch | 30 | 50 | 20 | 5 | 100 | 480 | 3.33 |
| 13 | Continuous | 30 | 50 | 20 | 1 | 50 | 900 | 0.63 |
| 14 | Continuous | 30 | 50 | 20 | 1 | 100 | 450 | 0.63 |
| 15 | Continuous | 30 | 50 | 20 | 1 | 150 | 300 | 0.63 |
| 16 | Continuous | 30 | 50 | 20 | 1 | 300 | 170 | 0.63 |
| [1] weight percentage, based on total weight of powder | | | | | | | | |

In all examples listed in Table 2 above a good quality of the produced granules is achieved in the batch process, in the stepped-batch process and in the continuous process. No large lumps of agglomerated powder or areas of dry, non-agglomerated powders are visible.

Examples 17 - 20 and Comparative Example A

a) Production of the Foam

Foam is prepared as in Examples 1-16 above except that an aqueous solution containing 1% (weight percent) of a binder is prepared in all examples and that a hydroxypropyl methylcellulose is used as a polymeric binder which is commercially available from The Dow Chemical Company under the Trademark METHOCEL E6PLV cellulose ether.

b) Powder Granulation

In Examples 17-20 powder with the composition indicated in Table 3 below is agglomerated as described for Examples 1-16 above.
In Comparative Example A powder is agglomerated according to conventional liquid spraying technology. The powder is loaded into the high-shear granulator and mixed for 1 minute to achieve a homogeneous and lump-free mix. An aqueous, 10 weight percent solution of METHOCEL E6PLV (trademark) is sprayed onto the moving powder bed at a spray rate of 75 grams per minute for approximately 700 seconds.

c) Tablet pressing

Tablets are prepared from the granulated powder of Examples 17-20 and Comparative Example A using a Manesty Beta rotary tablet press. The tablets exhibit a controlled release of the drug theophylline. The drug release of the prepared tablets are compared and represented graphically in Fig. 2.

Table 3

| Comparative Example | | 17 | 18 | 19 | 20 | A |
|---|---|---|---|---|---|---|
| Process type | | Foam, step batch[2] | Foam, continuous | Foam, continuous | Foam, batch[3] | Sprayed liquid |

(continued)

| Comparative Example | | 17 | 18 | 19 | 20 | A |
|---|---|---|---|---|---|---|
| Powder composition, average particle size less than 150 micrometers | K4MP (%)[1] | 25.9 | 25.9 | 25.9 | 25.9 | 25.9 |
| | FFL-316(%)[1] | 22.3 | 22.3 | 22.3 | 22.3 | 22.3 |
| | AvicelPH102 (%)[1] | 10.4 | 10.4 | 10.4 | 10.4 | 10.4 |
| | Theophylline (%)[1] | 41.4 | 41.4 | 41.4 | 41.4 | 41.4 |
| E6PLV solution | Binder conc., based on total solution (%) | 1 | 1 | 1 | 1 | 10 |
| | Addition rate (g/minute) | 75 g/min. | 75 g/min. | 300 g/min. | 300 g/min. | 75 g/min. |
| | Addition time (seconds) | 480 | 480 | 120 | 120 | 700 |
| | Binder level (%)[1] | 0.5 | 0.5 | 0.5 | 0.5 | 7.3 |
| [1] weight percentage, based on total weight of powder [2] Foam added in 8 equal portions to the powder [3] Foam added in one batch before granulator is set into operation | | | | | | |

In all examples 17 - 20 listed in Table 3 above a good quality of the produced granules is achieved in the batch process, in the stepped-batch process and in the continuous process. No large lumps of agglomerated powder or areas of dry, non-agglomerated powders are visible.

Fig. 2 illustrates that the drug release for different embodiments of carrying out the agglomeration process of the present invention, such as the addition of foam continuously, in one batch or in several batches or addition of the foam at different speeds, lead to pharmaceutical compositions with essentially the same drug release profile as a conventional agglomeration process wherein a corresponding liquid is sprayed on a corresponding powder. This is highly desirable since it allows the production of a pharmaceutical compositions with a predictable drug release. Moreover, it has been surprisingly found that in the process of the present invention much less binder is necessary than in a conventional process making use of sprayed liquid to obtain essentially the same drug release profile. This is illustrated by the binder level, based on the total weight of the powder. In Examples 17 to 20 foamed binder solutions comprising 1 weight percent of a binder are used in such an amount that a binder level of only about 0.5 percent results, based on the total weight of the powder. In Comparative Example A the sprayed liquid comprises 10 weight percent of a binder. The required binder level in Comparative Example A to achieve essentially the same drug release profile as in Examples 17 to 20 is about 7.3 percent, based on the total weight of the powder, as opposed to only about 0.5 percent in Examples 17 to 20.

Example 21 and Comparative Example B

a) Production of the Foam

Foam is prepared as in Examples 1-16 above, except that an aqueous solution containing 1 weight percent of a polymeric binder is prepared in all examples and that a hydroxypropyl methylcellulose is used as a polymeric binder which is commercially available from The Dow Chemical Company under the Trademark METHOCEL K3PLV.

b) Powder granulation

The foam produced in step a) is used for granulating 1500 g of a powder blend consisting of 44 percent ofNaproxyn Sodium, 25.5 percent of FFL-316 lactose, 0.5 percent of magnesium stearate and 30 percent of hydroxypropyl methylcellulose with a methoxyl substitution of 19-24 percent, a hydroxypropoxyl substitution of 7-12 percent and a viscosity of about 4,000 mPa·s, which is commercially available from The Dow Chemical Company under the Trademark METHOCEL K4MP CR. The granulation is carried out as described in step b) of Examples 1-16. The powder has an average particle size of less than about 200 micrometers. Foam is added continuously to the powder at a rate of 150 g/minute. The addition time is 120 seconds. The concentration of the METHOCEL K3PLV hydroxypropyl methylcellulose is 0.2 percent, based on the weight of the powder.

c) Further processing

The resultant granular material is dried on a tray in an oven at 110°F (43° C). A part of the dried granular material is sieved to the sizes indicated below. The non-sieved and sieved granular material is pressed to tablets using a Manesty Beta rotary tablet press.

Tablets are prepared in the same manner from a comparative, non-granulated powder which is used as a starting material in step b) above.

The hardness of the produced tablets is measured according to Strong-Cobb Units (SCU). SCU is known in the pharmaceutical art. The hardness, thickness and weight variation of the tablets are listed in Table 4 below.

Table 4

| (Comp.) Example | Particles | Hardness | | Thickness | | Weight Variation | |
|---|---|---|---|---|---|---|---|
| | | Mean [SCU] | Standard deviation | Mean [mm] | Standard deviation | Mean [mg] | Standard deviation |
| B | Non-granuated powder | 11.8 | 2.0 | 5.18 | 0.06 | 495 | 2.4 |
| 21-1 | Non-sieved granular material | 17.1 | 1.0 | 5.08 | 0.07 | 496.7 | 6.8 |
| 21-2 | Large sieved granules, 1000-1100 micrometers | 18.8 | 3.3 | 5.19 | 0.06 | 508.1 | 2.6 |
| 21-3 | Medium size sieved granules, 425-600 microns | 19.0 | 0.8 | 5.08 | 0.11 | 501 | 10.9 |
| 21-4 | small sieved granules, 180-260 microns | 18.9 | 1.6 | 5.12 | 0.07 | 502 | 7.7 |

The results in Table 4 show that harder tablets can be prepared from the granulated powder produced according to the process of the present invention than from corresponding non-granulated powder.

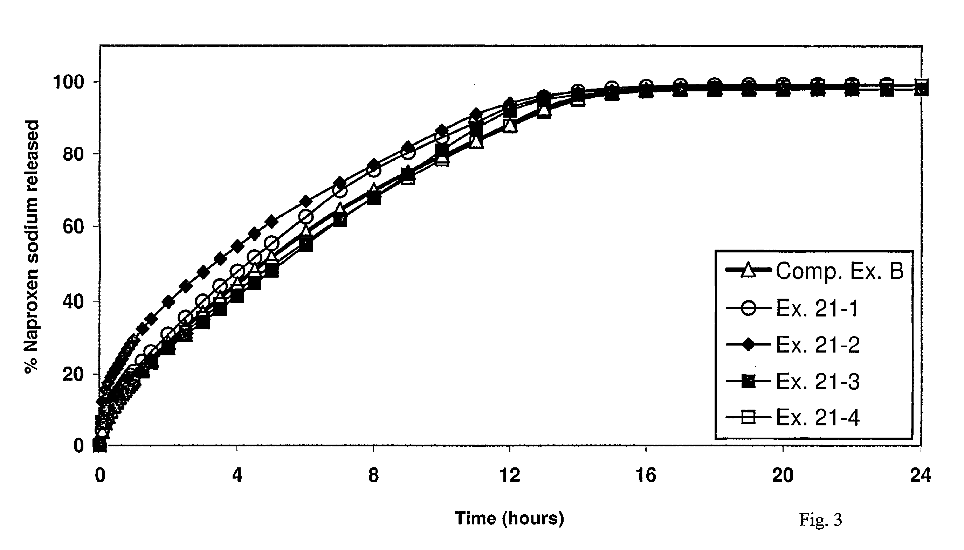

The drug release of Naproxyn sodium from the prepared tablets is shown in Figure 3. Fig. 3 illustrates that the drug release from the tablets produced according to the method of the present invention is essentially the same as a drug release profile from a tablet produced from non-agglomerated powder.

Examples 22-24

a) Production of the Foam

Foam is prepared as in Examples 1-16 above, except that an aqueous solution containing 2 weight percent of a polymeric binder is prepared in all examples and that a hydroxypropyl methylcellulose is used as a polymeric binder which is commercially available from The Dow Chemical Company under the Trademark METHOCEL K3PLV.

b) Powder granulation

The foam produced in step a) is used for granulating 1500 g of a powder blend consisting of 30.2 percent of POLYOX™ Water-Soluble Resin Coagulant NF, 40.2 percent of diphenhydramine HCl , 15.1 percent of Avicel PH102 microcrystalline cellulose, and 14.5 percent of unmilled dicalcium phosphate dihydrate. The POLYOX Water-Soluble Resin Coagulant NF is commercially available from Union Carbide Corporation, a subsidiary of The Dow Chemical Company and has a weight average molecular weight Mw of 5,000,000 and a viscosity of 5,500 -7,500 mPa.s, measured as a 1 weight percent solution at 25oC. The granulation is carried out as described in step b) of Examples 1-16). The powder has an average particle size of less than about 150 micrometers. Details on the granulation method are listed in Table 5 below.

Table 5

| Example | 22 | 23 | 24 |
|---|---|---|---|
| Mode of foam addition | Step batch, in two equal steps of 30 seconds each | Continuously over 60 seconds | added in one batch before granulator is set into operation |
| Foam addition rate (g/ minute) | 150 g/min. | 150 g/min. | 150 g/min. |
| g Foam added to 1.5 kg of powder | 150 g | 150 g | 150 g |
| Binder level (%), based on total weight of dry powder | 0.2% | 0.2% | 0.2% |
| Water level (%), based on total weight of dry powder | 9.8% | 9.8% | 9.8% |

In all examples listed in Table 5 above a good quality of the produced granules is achieved in the batch process, the stepped batch process and the continuous process. No large lumps of agglomerated powder or areas of dry, non-agglomerated powders are visible.

Examples 25-26 and Comparative Examples C and D

a) Production of the Foam

Foam is prepared as in Examples 1-16 above, except that an aqueous solution with the composition listed in Table 6 below is prepared and used for foam production. In Comparative Examples C and D an aqueous composition of 0.5 percent of sodium lauryl sulfate (SLS) surfactant is used. In Examples 25 and 26 an aqueous composition of 0.5 percent of sodium lauryl sulfate surfactant (SLS) and 2.0 percent of CELLOSIZE™ QP3L hydroxyethyl cellulose is used.

b) Powder granulation

The foam produced in step a) is used for granulating 1200 g of a powder blend consisting of 38 percent of sodium lauryl sulfate (SLS) surfactant, 40 percent of zeolite, type A with a particle size of less than 5 micrometers, 11 percent of sodium carbonate and 11 percent of an acrylic acid/maleic acid copolymer with a weight average molecular weight Mw of 70,000. The granulation is carried out as described in step b) of Examples 1-16. The powder has an average particle size of less than about 200 micrometers. Details on the powder granulation are listed in Table 6 below.

Table 6

| (Comparative) Example | C | D | 25 | 26 |
|---|---|---|---|---|
| Solution for foam preparation | 0.5 % SLS in water | 0.5 % SLS in water | 0.5 % SLS and 2.0 % CELLOSIZE™ QP3L in water | 0.5 % SLS and 2.0% CELLOSIZE™ QP3L in water |

(continued)

| (Comparative) Example | C | D | 25 | 26 |
|---|---|---|---|---|
| Mode of foam addition | Step batch, in 2 equal steps of 50 seconds each | Continuously over 95 seconds | Step batch, in 4 steps of 60 seconds each and 1 step of 5 seconds | Continuously over 258 seconds |
| Addition rate (g/minute) | 150 g/min. | 150 g/min. | 60 g/min. | 60 g/min. |
| g Foam added to 1.2 kg of powder | 250 g | 238 g | 245 g | 258 g |
| Polymeric binder level [1] | 0.0 % | 0.0 % | 0.4 % | 0.4 % |
| Water level[1] | 20.7 % | 19.7 % | 19.9 % | 21.0 % |
| [1], based on total weight of dry powder | | | | |

In Examples 25 and 26 listed in Table 6 above a good quality of the produced granules is achieved in the batch process and the continuous process. No large lumps of agglomerated powder or areas of dry, non-agglomerated powders are visible. The granules of Comparative Examples C and D are of insufficient quality. They are much more friable than the granules of Examples 25 and 26 and release a significant amount of dust.

Examples 27 - 33

a) Production of the Foam

Foam is prepared as in Examples 1-16 above, except that an aqueous solution with the composition listed in Table 7 below is prepared and used for foam production.

b) Powder granulation

The foam produced in step a) is used for granulating 1.5 kg of a powder blend consisting of 71.7 percent of the drug acetaminophen (60 mesh), 17.7 percent of FFL-316 fast flow lactose, 5.3 percent of Avicel PH 102 microcristalline cellulose and 5.3 percent of pregelatinized corn starch, commercially available from Colorcon (West Point, PA, USA) as Starch 1500.
The granulation is carried out as described in step b) of Examples 1-16. The powder has an average particle size of less than about 200 micrometers. Details on the powder granulation are listed in Table 7 below.

c) Tablet pressing

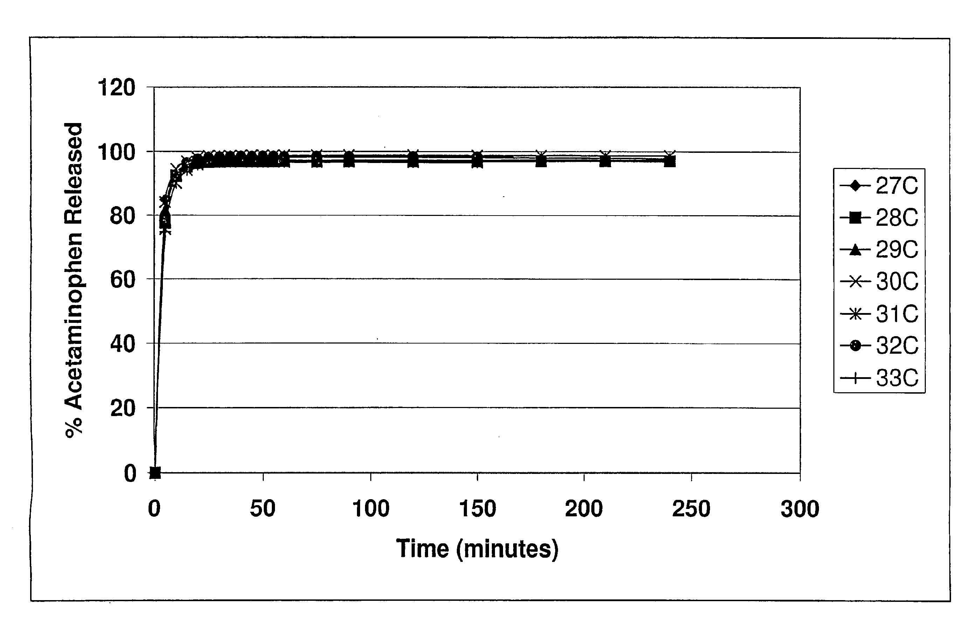

The granulated powders of Examples 27C - 33C are mixed with 3.1 percent of croscarmellose sodium and 0.5 percent of magnesium stearate, based on the dry powder blend used in paragraph b) of Examples 27-33 above. Tablets are pressed from the prepared mixtures using a Manesty Beta rotary tablet press. The tablets exhibit an immediate release of the drug acetaminophen. The drug releases of the prepared tablets are compared and represented graphically in Fig. 4.

Table 7

| Example | Process type | Solution for foam preparation | | Foam | | | Granulate | |
|---|---|---|---|---|---|---|---|---|
| | **Mode of foam addition** | **Type of binder** | **Binder concentration[1] (%)** | **Addition rate (g/ minute)** | **Addition time (seconds)** | **Foam added (g)** | **Binder level [3] (%)** | **Water level [3] (%)** |
| 27 SB | step batch, in 5 equal steps of 30 seconds each | PVP-K30 | 10 | 150 | 150 | 375 | 2.5 | 22.5 |
| 27 C | Continuous | PVP-K30 | 10 | 150 | 150 | 375 | 2.5 | 22.5 |
| 28 SB | step batch, in 5 steps of 30 seconds each and 1 step of 15 seconds | PVP-K30 | 2 | 150 | 165 | 413 | 0.6 | 27.0 |
| 28 C | Continuous | PVP-K30 | 2 | 150 | 165 | 413 | 0.6 | 27.0 |
| 29 SB | step batch, in 5 steps of 30 seconds each and 1 step of 15 seconds | PVP-K30 + 0.1% SLS[2] | 10 | 150 | 165 | 413 | 2.8 | 24.8 |
| 29 C | Continuous | PVP-K30 + 0.1% SLS[2] | 10 | 150 | 165 | 413 | 2.8 | 24.8 |
| 30 SB | step batch, in 5 steps of 30 seconds each and 1 step of 15 seconds | PVP-K30 + 0.1% SLS[2] | 2 | 150 | 165 | 413 | 0.6 | 27.0 |
| 30 C | Continuous | PVP-K30 + 0.1% SLS[2] | 2 | 150 | 150 | 375 | 0.5 | 24.5 |
| 31 SB | step batch, in 5 steps of 30 seconds each and 1 step of 15 seconds | PVP-K30 + 2% E3P[2] | 10 | 150 | 165 | 413 | 2.8 | 24.8 |
| 31 C | Continuous | PVP-K30 + 2% E3P[2] | 10 | 150 | 165 | 413 | 2.8 | 24.8 |

(continued)

| Example | Process type | Solution for foam preparation | | Foam | | | Granulate | |
|---|---|---|---|---|---|---|---|---|
| | Mode of foam addition | Type of binder | Binder concentration[1] (%) | Addition rate (g/ minute) | Addition time (seconds) | Foam added (g) | Binder level [3] (%) | Water level [3] (%) |
| 32 SB | step batch, in 5 steps of 30 seconds each and 1 step of 15 seconds | PVP-K30 + 10% E3P[2] | 2 | 150 | 165 | 413 | 0.6 | 27.0 |
| 32 C | Continuous | PVP-K30 + 10% E3P[2] | 2 | 150 | 165 | 413 | 0.6 | 27.0 |
| 33 SB | step batch, in 5 steps of 30 seconds each | E3P | 2 | 150 | 150 | 375 | 0.5 | 24.5 |
| 33 C | Continuous | E3P | 2 | 150 | 150 | 375 | 0.5 | 24.5 |
| [1] weight percentage, based on total weight of solution [2] weight percentage, based on weight of PVP-K30 [3] weight percentage, based on total weight of powder | | | | | | | | |

## Claims

1. A process for dispersing a fluid in solid particles comprising the steps of a) contacting a gas with a fluid composition comprising i) from 0.01 to 30 weight percent of a polymer having a weight average molecular weight of at least 10000 and ii) from 99.99 to 70 weight percent of a liquid diluent, based on the total weight of the polymer i) and the liquid diluent ii), to produce a foam, and b) contacting the produced foam with solid particles of an average size of less than 2500 micrometers, the weight ratio between the foam and the solid particles being from 1 : 20 to 1 : 0.2 wherein the fluid composition comprises a drug or the solid particles comprise a drug or the fluid composition and the solid particles comprise a drug.

2. A process for dispersing a fluid in solid particles comprising the steps of a) contacting a gas with a fluid composition comprising i) from 0.01 to 30 weight percent of a polymer having a weight average molecular weight of at least 10000 and ii) from 99.99 to 70 weight percent of a liquid diluent, based on the total weight of the polymer i) and the liquid diluent ii), to produce a foam, and b) contacting the produced foam with solid particles of an average size of less than 2500 micrometers, the weight ratio between the foam and the solid particles being from 1 : 20 to 1 : 0.2 wherein the solid particles are pharmaceutical excipients.

3. The process of Claim 1 or Claim 2 wherein in step b) the solid particles are a least partially coated with the foam.

4. The process of Claim 1 or Claim 2 wherein solid particles are agglomerated.

5. The process of Claim 4 wherein the fluid composition comprises a binder for the solid particles and in step b) a granular material is produced by contacting the foam with the solid particles and agglomerating particles.

6. The process of Claim 5 wherein the produced granular material is dried and processed to a material of an average particle size of from 10 to 10,000 micrometers.

7. The process of any one of Claims 1 to 6 wherein the solid particles are a powder of an average particle size of less than 1000 micrometers.

8. The process of any one of Claims 1 to 7 wherein the polymer i) is gum arabic, xanthan gum, gum karaya, gum tragacanth, gum ghatti, guar gum, an exudate gum, a seaweed gum, a seed gum, a microbial gum, carrageenan, dextran, gelatin, an alginate, a pectin, a starch, a polysaccharide, a starch derivative, a guar derivative, a xanthan derivative, a polyalkylene oxide; a homo- or copolymer of ethylene imine, acrylic acid or a salt thereof, acrylamide, vinylalcohol, vinylacetate, vinylpyrrolidone, vinyloxazolidone, vinylmethyloxazolidone, ethylene sulfonic acid, vinylamine, vinylpyrridine, an alkylglycol or an oxyethylene alkylether, of a blend of two or more of said polymers.

9. The process of Claim 8 wherein the polymer i) is a cellulose ether or a cellulose ester.

10. The process of Claim 9 wherein the gas is contacted with an aqueous composition comprising from 0.01 to 30 weight percent of a water-soluble cellulose ether, based on the total weight of the cellulose ether and water.

11. The process of any one of Claims 1 to 10 wherein the fluid composition and the foam do not comprise a surfactant other than the polymer i).

12. The process of any one of Claims 1 to 11 wherein the foam is a water-based air foam.

13. The process of any one of Claims 1 to 12 wherein the pharmaceutical excipients are lactose, dicalcium phosphate or microcrystalline cellulose.

14. The process of any one of Claims 1 to 13 wherein step b) is carried out in a mixing device which is set into operation after the solid particles and the foam have been fed to the mixing device.

15. The process of Claim 14 wherein the solid particles are fed to the mixing device, foam is placed on top of the solid particles and the mixing device is subsequently set into operation.

16. The process of any one of Claims 1 to 13 wherein step b) is carried out in the sub-steps of bi) feeding the solid particles and a foam portion to a mixing device, bii) setting the mixing device into operation to disperse the foam portion in the solid particles, biii) stopping the operation of the mixing device, biv) feeding an additional foam portion to the mixing device, bv) setting the mixing device into operation to disperse the foam portion in the solid particles, bvi) stopping the operation of the mixing device, and repeating steps biv) to bvi) several times.

17. The process of any one of Claims 1 to 13 wherein in step b) the solid particles are fed to a mixing device and foam is added continuously or in portions to the mixing device while the mixing device is in operation.

18. The process of any one of Claims 1 to 17 wherein step b) is conducted in a low shear mixing device, a fluidized bed granulator, a roller compactor or a spray dryer.

19. The process of any one of Claims 1 to 18 wherein a pharmaceutical composition with a controlled drug release is prepared.

20. A process for preparing a tablet wherein a granular material is produced according to the process of any one of Claims 4 to 19 and pressed to a tablet.


**Patentansprüche**

1. Verfahren zum Dispergieren eines Fluids in Feststoffpartikeln, umfassend die Schritte a) Inkontaktbringen eines Gases mit einer Fluidzusammensetzung, umfassend i) von 0,01 bis 30 Gewichtsprozent eines Polymers mit einem Molekulargewicht im Gewichtsmittel von wenigstens 10000 und ii) von 99,99 bis 70 Gewichtsprozent eines flüssigen Verdünnungsmittels, bezogen auf das Gesamtgewichts des Polymers i) und des flüssigen Verdünnungsmittels ii), zur Herstellung eines Schaums und b) Inkontaktbringen des hergestellten Schaums mit Feststoffpartikeln einer durchschnittlichen Größe von weniger als 2500 Mikrometer, wobei das Gewichtsverhältnis zwischen dem Schaum und den Feststoffpartikeln von 1:20 bis 1:0,2 ist, wobei die Fluidzusammensetzung ein Arzneimittel umfasst oder die Feststoffpartikel ein Arzneimittel umfassen oder die Fluidzusammensetzung und die Feststoffpartikel ein Arzneimittel umfassen.

**2.** Verfahren zum Dispergieren eines Fluids in Feststoffpartikeln, umfassend die Schritte a) Inkontaktbringen eines Gases mit einer Fluidzusammensetzung, umfassend i) von 0,01 bis 30 Gewichtsprozent eines Polymers mit einem Molekulargewicht im Gewichtsmittel von wenigstens 10000 und ii) von 99,99 bis 70 Gewichtsprozent eines flüssigen Verdünnungsmittels, bezogen auf das Gesamtgewichts des Polymers i) und des flüssigen Verdünnungsmittels ii), zur Herstellung eines Schaums und b) Inkontaktbringen des hergestellten Schaums mit Feststoffpartikeln einer durchschnittlichen Größe von weniger als 2500 Mikrometer, wobei das Gewichtsverhältnis zwischen dem Schaum und den Feststoffpartikeln von 1:20 bis 1:0,2 ist, wobei die Feststoffpartikel pharmazeutische Hilfsstoffe sind.

**3.** Verfahren nach Anspruch 1 oder 2, wobei in Schritt b) die Feststoffpartikel wenigstens teilweise mit dem Schaum beschichtet werden.

**4.** Verfahren nach Anspruch 1 oder 2, wobei die Feststoffpartikel agglomeriert werden.

**5.** Verfahren nach Anspruch 4, wobei die Fluidzusammensetzung ein Bindemittel für die Feststoffpartikel umfasst und in Schritt b) ein gekörntes Material durch Inkontaktbringen des Schaums mit den Feststoffpartikeln und agglomerierenden Partikeln hergestellt wird.

**6.** Verfahren nach Anspruch 5, wobei das hergestellte gekörnte Material getrocknet wird und zu einem Material mit einer durchschnittlichen Partikelgröße von 10 bis 10.000 Mikrometern verarbeitet wird.

**7.** Verfahren nach Anspruch 1 bis 6, wobei die Feststoffpartikel ein Pulver mit einer durchschnittlichen Partikelgröße von weniger als 1000 Mikrometern sind.

**8.** Verfahren nach einem der Ansprüche 1 bis 7, wobei das Polymer i) Gummi arabicum, Xanthangummi, Karayagummi, Tragantgummi, Ghattigummi, Guargummi, ein Exudatgummi, ein Meeresalgengummi, ein Saatgummi, ein mikrobieller Gummi, Carragenan, Dextran, Gelatine, ein Alginat, ein Pectin, eine Stärke, ein Polysaccharid, ein Stärkederivat, ein Guarderivat, ein Xanthanderivat, ein Polyalkylenoxid; ein Homo- oder Copolymer von Ethylenimin, Acrylsäure oder ein Salz davon, Acrylamid, Vinylalkohol, Vinylacetat, Vinylpyrrolidon, Vinyloxazolidon, Vinylmethyloxazolidon, Ethylensulfonsäure, Vinylamin, Vinylpyridin, ein Alkylglycol oder ein Oxyethylenalkylether oder ein Gemisch von zwei oder mehreren dieser Polymere ist.

**9.** Verfahren nach Anspruch 8, wobei das Polymer i) ein Celluloseether oder ein Celluloseester ist.

**10.** Verfahren nach Anspruch 9, wobei das Gas in Kontakt gebracht wird mit einer wässrigen Zusammensetzung, umfassend von 0,01 bis 30 Gewichtsprozent eines wasserlöslichen Celluloseethers bezogen auf das Gesamtgewicht des Celluloseethers und Wasser.

**11.** Verfahren nach einem der Ansprüche 1 bis 10, wobei die Fluidzusammensetzung und der Schaum kein anderes oberflächenaktives Mittel als das Polymer i) umfassen.

**12.** Verfahren nach einem der Ansprüche 1 bis 11, wobei der Schaum ein auf Wasser basierender Luftschaum ist.

**13.** Verfahren nach einem der Ansprüche 1 bis 12, wobei die pharmazeutischen Hilfsmittel Lactose, Dicalciumphosphat oder mikrokristalline Cellulose sind.

**14.** Verfahren nach einem der Ansprüche 1 bis 13, wobei Schritt b) ausgeführt wird in einer Mischungsvorrichtung, welche in Betrieb genommen wird, nachdem die Feststoffpartikel und der Schaum der Mischungsvorrichtung zugeführt worden sind.

**15.** Verfahren nach Anspruch 14, wobei die Feststoffpartikel der Mischungsvorrichtung zugeführt werden, Schaum auf den Feststoffpartikeln platziert wird und die Mischungsvorrichtung anschließend in Betrieb genommen wird.

**16.** Verfahren nach einem der Ansprüche 1 bis 13, wobei Schritt b) ausgeführt wird in den Unterschritten bi) Zuführen der Feststoffpartikel und eines Schaumanteils zu einer Mischungsvorrichtung, bii) Inbetriebnahme der Mischungsvorrichtung zum Dispergieren des Schaumanteils in den Feststoffpartikeln, biii) Anhalten des Betriebs der Mischungsvorrichtung, biv) Zuführen eines weiteren Schaumanteils zu der Mischungsvorrichtung, bv) Inbetriebnahme der Mischungsvorrichtung zum Dispergieren des Schaumanteils in den Feststoffpartikeln, bvi) Anhalten des Betriebs der Mischungsvorrichtung und mehrmalige Wiederholung der Schritte biv) bis bvi).

**17.** Verfahren nach einem der Ansprüche 1 bis 13, wobei in Schritt b) die Feststoffpartikel einer Mischungsvorrichtung zugeführt werden und Schaum kontinuierlich oder in Teilen zu der Mischungsvorrichtung zugegeben wird, während die Mischungsvorrichtung in Betrieb ist.

**18.** Verfahren nach einem der Ansprüche 1 bis 17, wobei Schritt b) durchgeführt wird in einer Mischungsvorrichtung mit niedriger Scherbeanspruchung, einem Fließbettgranulator, einer Rollenpresse oder einem Sprühtrockner.

**19.** Verfahren nach einem der Ansprüche 1 bis 18, wobei eine pharmazeutische Zusammensetzung mit einer kontrollierten Arzneimittelfreisetzung hergestellt wird.

**20.** Verfahren zur Herstellung einer Tablette, wobei ein körnchenförmiges Material gemäß dem Verfahren nach einem der Ansprüche 4 bis 19 hergestellt wird und zu einer Tablette verpresst wird.

**Revendications**

**1.** Procédé de dispersion d'un fluide dans des particules solides, comportant les étapes suivantes :

a) mettre un gaz en contact avec une composition de fluide comprenant :

i) de 0,01 à 30 % en poids d'un polymère qui présente une masse molaire moyenne en poids d'au moins 10 000,
ii) et de 99,99 à 70 % en poids d'un diluant liquide,

en pourcentages rapportés au poids total du polymère (i) et du diluant liquide (ii), de manière à produire une mousse,
b) et mettre la mousse ainsi produite en contact avec des particules solides dont la taille moyenne vaut moins de 2500 micromètres, en un rapport pondéral entre la mousse et les particules solides de 1/20 à 1/0,2,

dans lequel procédé la composition de fluide comprend un médicament, les particules solides comprennent un médicament, ou la composition de fluide et les particules solides comprennent un médicament.

**2.** Procédé de dispersion d'un fluide dans des particules solides, comportant les étapes suivantes :

a) mettre un gaz en contact avec une composition de fluide comprenant :

i) de 0,01 à 30 % en poids d'un polymère qui présente une masse molaire moyenne en poids d'au moins 10 000,
ii) et de 99,99 à 70 % en poids d'un diluant liquide,

en pourcentages rapportés au poids total du polymère (i) et du diluant liquide (ii), de manière à produire une mousse,
b) et mettre la mousse ainsi produite en contact avec des particules solides dont la taille moyenne vaut moins de 2500 micromètres, en un rapport pondéral entre la mousse et les particules solides de 1/20 à 1/0,2,

dans lequel procédé les particules solides constituent des excipients pharmaceutiques.

**3.** Procédé conforme à la revendication 1 ou 2, dans lequel, dans l'étape (b), on fait en sorte que les particules solides soient au moins partiellement enrobées avec la mousse.

**4.** Procédé conforme à la revendication 1 ou 2, dans lequel on fait s'agglomérer les particules solides.

**5.** Procédé conforme à la revendication 4, dans lequel la composition de fluide comprend un agent liant les particules solides, et dans l'étape (b), on produit un matériau granulaire en mettant la mousse en contact avec les particules solides et en faisant s'agglomérer ces particules.

**6.** Procédé conforme à la revendication 5, dans lequel on fait sécher le matériau granulaire produit et on le traite pour en faire un matériau en particules d'une taille moyenne de 10 à 10 000 micromètres.

**7.** Procédé conforme à l'une des revendications 1 à 6, dans lequel les particules solides constituent une poudre dont les particules présentent une taille moyenne inférieure à 1 000 micromètres.

**8.** Procédé conforme à l'une des revendications 1 à 7, dans lequel le polymère (i) est de la gomme arabique, de la gomme xanthane, de la gomme karaya, de la gomme adragante, de la gomme ghatti, de la gomme de guar, une gomme d'exsudat, une gomme d'algues, une gomme de graines, une gomme microbienne, du carraghénane, du dextrane, de la gélatine, un alginate, une pectine, un amidon, un polysaccharide, un dérivé d'amidon, un dérivé de guar, un dérivé de xanthane, un poly(oxyalkylène), un homopolymère ou un copolymère d'éthylène-imine, d'acide acrylique ou de l'un de ses sels, d'acrylamide, d'alcool vinylique, d'acétate de vinyle, de vinyl-pyrrolidone, de vinyl-oxazolidone, de vinyl-méthyl-oxazolidone, d'acide éthylène-sulfonique, de vinyl-amine ou de vinyl-pyridine, un alkyl-glycol ou un éther alkylique d'oxyéthylène, ou un mélange de deux de ces polymères ou plus.

**9.** Procédé conforme à la revendication 8, dans lequel le polymère (i) est un éther de cellulose ou un ester de cellulose.

**10.** Procédé conforme à la revendication 9, dans lequel on met le gaz en contact avec une composition aqueuse comprenant de 0,01 à 30 % en poids d'un éther de cellulose hydrosoluble, pourcentage rapporté au poids total d'eau et d'éther de cellulose.

**11.** Procédé conforme à l'une des revendications 1 à 10, dans lequel la composition de fluide et la mousse ne contiennent pas d'autre tensio-actif que le polymère (i).

**12.** Procédé conforme à l'une des revendications 1 à 11, dans lequel la mousse est une mousse d'air à base d'eau.

**13.** Procédé conforme à l'une des revendications 1 à 12, dans lequel les excipients pharmaceutiques sont du lactose, du phosphate dicalcique ou de la cellulose microcristalline.

**14.** Procédé conforme à l'une des revendications 1 à 13, dans lequel on réalise l'étape (b) dans un dispositif mélangeur que l'on fait fonctionner après y avoir introduit les particules solides et la mousse.

**15.** Procédé conforme à la revendication 14, dans lequel on introduit les particules solides dans le dispositif mélangeur, on met la mousse par-dessus ces particules solides et l'on fait ensuite fonctionner le dispositif mélangeur.

**16.** Procédé conforme à l'une des revendications 1 à 13, dans lequel on effectue l'étape (b) en les sous-étapes suivantes :

bi) introduire dans un dispositif mélangeur les particules solides et une portion de la mousse,
bii) faire fonctionner le dispositif mélangeur, afin de disperser la portion de mousse dans les particules solides,
biii) interrompre le fonctionnement du dispositif mélangeur,
biv) introduire dans le dispositif mélangeur une portion supplémentaire de mousse,
bv) faire fonctionner le dispositif mélangeur, afin de disperser cette portion de mousse dans les particules solides,
bvi) interrompre le fonctionnement du dispositif mélangeur,

et répéter à plusieurs reprises les étapes (biv) à (bvi).

**17.** Procédé conforme à l'une des revendications 1 à 13, dans lequel, dans l'étape (b), on introduit les particules solides dans un dispositif mélangeur et l'on ajoute la mousse, en continu ou par portions, dans le dispositif mélangeur pendant que celui-ci fonctionne.

**18.** Procédé conforme à l'une des revendications 1 à 17, dans lequel on réalise l'étape (b) dans un mélangeur à faible cisaillement, un granulateur à lit fluidisé, un appareil de compaction à rouleau ou un appareil de séchage par pulvérisation.

**19.** Procédé conforme à l'une des revendications 1 à 18, dans lequel on prépare une composition pharmaceutique à libération retardée du médicament.

**20.** Procédé de préparation d'un comprimé, dans lequel on produit, en opérant selon un procédé conforme à l'une des revendications 4 à 19, un matériau granulaire que l'on presse pour en faire un comprimé.

Fig. 1

Fig. 2

Fig. 3

Time (hours)

% Naproxen sodium released

Comp. Ex. B
Ex. 21-1
Ex. 21-2
Ex. 21-3
Ex. 21-4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3725556 A
- US 4476145 A
- GB 2355008 A
- EP 0504870 B
- US 4645812 A
- US 5026735 A

**Non-patent literature cited in the description**

- Granulation and Scale-Up Issues in Solid Dosage Form Development. *American Pharmaceutical Review,* 2000, vol. 3 (4), 33-36
- **J. F. FRY ; R. J. FRENCH.** A mechanical foam-generator for use in laboratories. *J. Appl. Chem.,* 1951, vol. 1, 425-429